# EUROPEAN PATENT APPLICATION

(11) **EP 1 563 863 A1**
(43) Date of publication of application: **17.08.2005**
(21) Application number: 03758810.0
(22) Date of filing: 23.10.2003
(51) Int. Cl.: A61M 5/31, A61M 39/10, A61M 39/20

(54) **METHOD OF PRODUCING SYRINGE, CAP, AND PREFILLED SYRINGE**

(30) Priority: 24.10.2002 JP 2002310199; 06.10.2003 JP 2003347502; 06.10.2003 JP 2003347503
(71) Applicant: TERUMO KABUSHIKI KAISHA, Tokyo 151-0072 (JP)
(72) Inventor: KITO, Hideaki c/o Terumo kabushiki kaisha, Yamanashi 409-3853 (JP); TACHIKAWA, Kouichi c/o Terumo kabushiki kaisha, Yamanashi 409-3853 (JP)
(74) Representative: TBK-Patent
(86) International application number: PCT/JP2003/013540
(87) International publication number: WO 2004/037329

(57) **Abstract**

A first female connector (9) and a second female connector that has on its outer periphery a screw portion engaging a female screw can be alternatively connected to the top of a syringe (1). A mouth portion (22) with a smaller diameter than a barrel portion of an outer hollow-cylinder main body (21) is provided at the top of an outer hollow-cylinder. A lock adapter (23) is provided on the outer periphery of the mouth portion. The lock adapter (23) is rotatable about the mouth portion and axially movable in an axial direction of the mouth portion. The lock adapter can be moved to a position at the base of the mouth portion where the operation of fitting a male taper portion (222) formed at the top of the mouth portion into a bore portion (91) of the first female connector is not interrupted. The lock adapter can be fixed to the mouth portion at the position to which the adapter is moved.

## Description

### Technical Field

The present invention relates to a syringe, a cap, and a method of producing a prefilled syringe provided with said cap.

### Background Art

In medical sites, there are cases where various medical implements such as syringes, injection needles, catheters, and infusion sets are used in connection with each other.

Methods for the connection include, for example, a method in which a male taper portion possessed by a male connector provided at an end portion of one medical implement is fitted into a bore portion of a female connector provided at an end portion of the other medical implement.

In addition, the female connectors include one which is provided in its outer peripheral surface with a screw groove (a female-side screw engagement portion), and, correspondingly, the male connectors include one which is provided, at the outer peripheral portion of the male taper portion, with a connection member formed with a screw thread (a male-side screw engagement portion) for engagement with the screw groove of the female connector. This combination promises a firmer connection between the female connector and the male connector (refer to, for example, Japanese Utility Model Publication No. Hei 2-193).

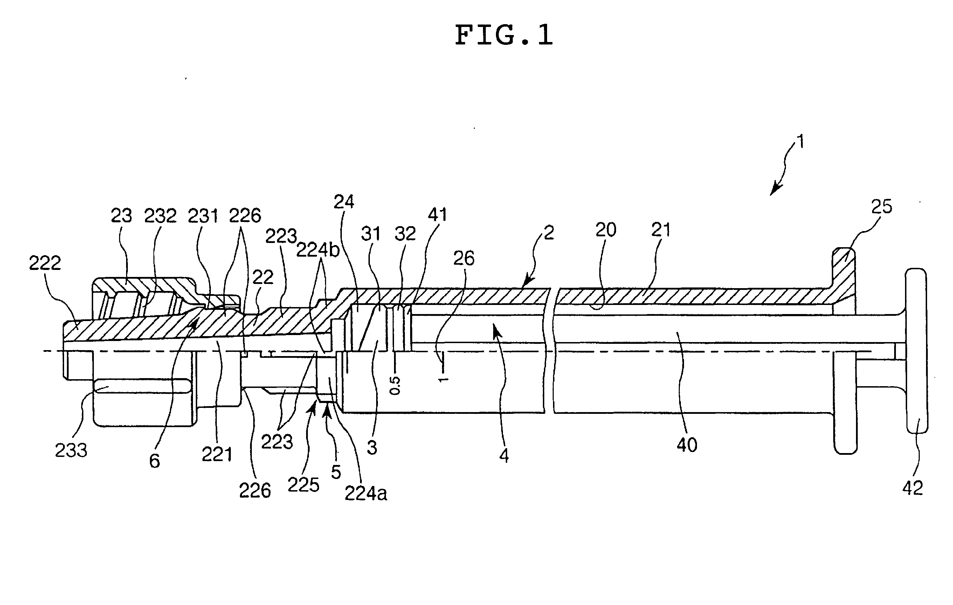

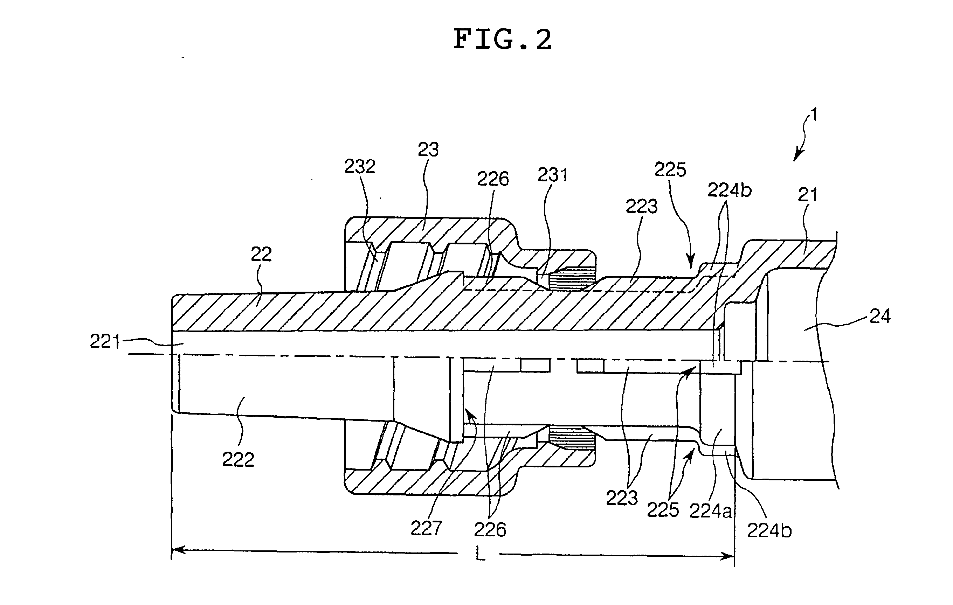

However, in the female connector configured as above, the connection member is fixed and, therefore, cannot be relatively moved along the axial direction of the male connector (see FIG. 1 of Japanese Utility Model Publication No. Hei 2-193), or, alternatively, the connection member can be relatively moved, to some extent, along the axial direction of the male connector (see FIG. 2 of Japanese Utility Model Publication No. Hei 2-193). Therefore, when it is intended to connect a female connector not formed with any screw portion on its outer peripheral surface to the male taper portion, the connection member obstructs the connecting operation, so that the connection cannot be performed assuredly.

As a male connector for solving this problem, there is a male connector in which a connection member can be relatively moved along the axial direction of a male connector so that a male taper portion as a whole can be exposed (refer to, for example, Japanese Patent Laid-open No. Hei 7-148271).

In this male connector, the connection member is made to be relatively movable along the axial direction of the male connector by a predetermined operation, specifically, by relatively rotating the connection member (lock nut portion 3) by a predetermined angle about the male taper portion (male lure portion 5). Then, by moving the connection member away from the male taper portion, the male taper portion as a whole can be exposed. Incidentally, the symbols used here are the symbols as used in Japanese Patent Laid-open No. Hei 7-148271.

In such a male connector, however, the connection member cannot be relatively moved along the axial direction of the male connector unless the predetermined operation is carried out, and, therefore, it is extremely intricate to expose the male taper portion.

Besides, the connection member is not fixed in the condition where the male taper portion is exposed. Therefore, at the time of connecting a female connector not formed with any screw groove in its outer peripheral surface to the male taper portion, the connection member may move along the axial direction of the female connector, thereby obstructing the connecting operation.

Meanwhile, among medical implements, syringes are used particularly frequently and are required to be capable of being assuredly connected to various medical implements.

In addition, there have been known prefilled syringes in which a sterilized syringe (container) is filled with a liquid preparation in a sterile condition.

As one of such prefilled syringes, there has been disclosed a prefilled syringe which has a lock adapter at an outer peripheral portion of a liquid discharge portion (mouth portion) thereof (refer to, for example, Japanese Patent Laid-open No. Hei 8-126701).

This lock adapter is provided at its inner peripheral surface with a screw portion, which is for screw engagement with a screw portion of a connector (connection means) to which, for example, a tube (tubular body) is connected. The screw engagement enables the prefilled syringe to be connected to the connector. Then, the liquid preparation with which the syringe is prefilled can be fed into the tube.

Meanwhile, the prefilled syringe provided with the lock adapter has had the problem that there is no cap for hermetically closing (sealing) the liquid discharge portion. Therefore, at the time of connecting the prefilled syringe to the connector, the liquid discharge portion would be exposed to the atmosphere (air) for a long time, whereby the liquid discharge portion may be polluted with bacteria.

### Disclosure of Invention

It is a first object of the present invention to provide a syringe which enables easy and assured connection thereof with both a female connector having a female-side screw engagement portion on its outer peripheral surface and a female connector not having the female-side screw engagement portion.

The first object of the present invention is achieved by the present invention as set forth in the following paragraphs (1) to (12).
(1) A syringe having a distal end portion to which a first female connector and a second female connector provided on an outer peripheral surface thereof with a female-side screw engagement portion can be alternatively connected, the syringe comprising:
   a mouth portion which is provided at a distal end portion thereof with a male taper portion to be fitted in a bore portion of the first female connector and a bore portion of the second female connector and which is provided therein with a passage for permitting a liquid to pass therethrough; and
   a lock adapter provided at an outer peripheral portion of the mouth portion, the lock adapter being relatively movable in the axial direction of the mouth portion and being provided on an inner peripheral surface thereof with a male-side screw engagement portion for making screw engagement with the female-side screw engagement portion; wherein
   the lock adapter can be retracted to a retraction position on the proximal end side at the time of an operation of fitting the male taper portion into the bore portion of the first female connector.
(2) A syringe having a distal end portion to which a first female connector and a second female connector provided on an outer peripheral surface thereof with a female-side screw engagement portion can be alternatively connected, the syringe comprising:
   a mouth portion which is provided at a distal end portion thereof with a male taper portion to be fitted in a bore portion of the first female connector and a bore portion of the second female connector and which is provided therein with a passage for permitting a liquid to pass therethrough; and
   a lock adapter provided at an outer peripheral portion of the mouth portion, the lock adapter being relatively rotatable about the mouth portion, being relatively movable along the axial direction of the mouth portion, and being provided on an inner peripheral surface thereof with a male-side screw engagement portion for making screw engagement with the female-side screw engagement portion; wherein
   the lock adapter can be retracted to a retraction position on the proximal end side at the time of an operation of fitting the male taper portion into the bore portion of the first female connector.
(3) A syringe as set forth in the above paragraph (2), having a rotation permitting position where the lock adapter is relatively rotatable about the mouth portion, on the distal end side relative to the retraction position.
(4) A syringe as set forth in any of the above paragraphs (1) to (3), wherein the lock adapter is fixed to the mouth portion when located in the retraction position.
(5) A syringe as set forth in any of the above paragraphs (1) to (4), wherein the male taper portion protrudes beyond the distal end of the lock adapter by not less than 7.5 mm when the lock adapter is located in the retraction position.
(6) A syringe as set forth in any of the above paragraphs (1) to (5), having a distal end side fixation position where the lock adapter is fixed to the mouth portion when the lock adapter is located on the distal end side of the mouth portion.
(7) A syringe as set forth in the above paragraph (6), having a rotation permitting position where the lock adapter is relatively rotatable about the mouth portion, on the distal end side relative to the retraction position, wherein
   the distal end side fixation position is located on the mouth portion distal end side relative to the rotation permitting position.
(8) A syringe as set forth in the above paragraph (6) or (7), wherein the male taper portion protrudes beyond the distal end of the lock adapter by not less than 2.1 mm when the lock adapter is located in the distal end side fixation position.
(9) A syringe as set forth in any of the above paragraphs (1) to (8), wherein the lock adapter is relatively movable by not less than 5.4 mm along the axial direction of the mouth portion.
(10) A syringe as set forth in any of the above paragraphs (1) to (9), wherein the inside diameter (on average) of the mouth portion is not less than 1.2 mm.
(11) A syringe as set forth in any of the above paragraphs (1) to (10), wherein the length of the mouth portion is in the range of 16 to 20 mm.
(12) A syringe as set forth in any of the above paragraphs (1) to (11), comprising an outer hollow cylinder provided at a distal end portion thereof with the mouth portion and the lock adapter, and a gasket slidable in said outer hollow cylinder, wherein
   the volume of a space defined by the outer hollow cylinder and the gasket when the gasket is located at the distal end of the inside of the outer hollow cylinder is not more than 0.1 mL.
   It is a second object of the present invention to provide a cap which is capable of being mounted to a prefilled syringe having a lock adapter and which makes it possible to keep in a sterile condition the surroundings of a mouth portion of a syringe outer hollow cylinder after a sterilizing treatment. It is also the second object of the present invention to provide a method of producing a prefilled syringe in which the cap is used.
   The second object of the present invention is achieved by the present invention as set forth in the following paragraphs (13) to (16).
(13) A cap to be mounted to a mouth portion of a syringe outer hollow cylinder, the syringe outer hollow cylinder comprising the mouth portion projectingly formed at the distal end of the syringe outer hollow cylinder and provided at a distal end portion thereof with a male taper portion, and a lock adapter provided at an outer peripheral portion of the mouth portion, the lock adapter being relatively movable along the axial direction of the mouth portion and being provided on an inner peripheral surface thereof with a male-side screw engagement portion, the cap comprising:
   a bottomed hollow-cylindrical cap main body comprising a bore portion, and a female-side screw engagement portion formed on an outer peripheral portion of the cap main body for screw engagement with the male-side screw engagement portion; and
   a packing formed of an elastic material and provided in the bore portion of the cap main body; wherein
   at least a part of the inner peripheral surface of the bore portion makes close contact with the male taper portion over the entire circumference when the cap main body is mounted to the mouth portion.
(14) A cap as set forth in the above paragraph (13), wherein when the inner peripheral surface of the bore portion makes close contact with the male taper portion, the packing is clamped between an end face of the bore portion and the distal end of the mouth portion to seal the mouth portion in a liquid-tight manner.
(15) A method of producing a prefilled syringe comprising: a syringe outer hollow cylinder having a mouth portion projectingly formed at the distal end of the syringe outer hollow cylinder and provided at a distal end portion thereof with a male taper portion, and a lock adapter provided at an outer peripheral portion of the mouth portion, the lock adapter being relatively movable along the axial direction of the mouth portion and being provided on an inner peripheral surface thereof with a male-side screw engagement portion; a cap as set forth in the above paragraph (13) or (14) which is mounted to the mouth portion; and a liquid preparation filling the syringe outer hollow cylinder; the method comprising the steps of:
   sterilizing the syringe outer hollow cylinder and the cap;
   then mounting the cap to the mouth portion in a sterile environment; and
   thereafter feeding the liquid preparation into the syringe outer hollow cylinder in a sterile environment.
(16) A method of producing a prefilled syringe comprising: a syringe outer hollow cylinder having a mouth portion projectingly formed at the distal end of the syringe outer hollow cylinder and provided at a distal end portion thereof with a male taper portion, and a lock adapter provided at an outer peripheral portion of the mouth portion, the lock adapter being relatively movable along the axial direction of the mouth portion and being provided on an inner peripheral surface thereof with a male-side screw engagement portion; a cap as set forth in the above paragraphs (13) or (14) which is mounted to the mouth portion; and a liquid preparation filling the syringe outer hollow cylinder; the method comprising the steps of:
   mounting the cap to the mouth portion and performing sterilization under this condition; and
   thereafter feeding the liquid preparation into the syringe outer hollow cylinder in a sterile environment.

### Brief Description of Drawings

FIG. 1 is a vertical semi-sectional view of a first embodiment of a syringe according to the present invention.
FIG. 2 is a vertical partly sectional view of a distal end portion of the syringe shown in FIG. 1 (showing the condition where a lock adapter is located in a rotation permitting position).
FIG. 3 is a vertical partly sectional view of the distal end portion of the syringe shown in FIG. 1 (showing the condition where the lock adapter is located in a retraction position and a first female connector is connected).
FIG. 4 is a vertical partly sectional view of the distal end portion of the syringe shown in FIG. 1 (showing the condition where the lock adapter is located in a distal end side fixation position and a second connector is connected).
FIG. 5 is a vertical semi-sectional view of a second embodiment of the syringe according to the present invention.
FIG. 6 is a vertical partly sectional view of the distal end portion of the syringe shown in FIG. 5.
FIG. 7 is a vertical partly sectional view of the distal end portion of the syringe shown in FIG. 5 (showing the condition where a lock adapter is located in a retraction position and a first female connector is connected).
FIG. 8 is a vertical partly sectional view of the distal end portion of the syringe shown in FIG. 5 (showing the condition where the lock adapter is located in a distal end side fixation position and a second female connector is connected).
FIG. 9 is a sectional view along line A-A of FIG. 6.
FIG. 10 is a vertical semi-sectional view of an embodiment of a cap according to the present invention.
FIG. 11 is a vertical semi-sectional view of an embodiment of the cap according to the present invention.
FIG. 12 is a side view of a prefilled syringe in the condition where the cap shown in FIG. 10 is mounted.
FIG. 13 is a vertical partly sectional view of the prefilled syringe.

### Best Mode for Carrying out the Invention

Now, the syringe, the cap, and the method of producing a prefilled syringe provided with the cap according to the present invention will be described in detail below, based on the preferred embodiments shown in the accompanying drawings.

First, the syringe according to the present invention will be described.

### <First Embodiment>

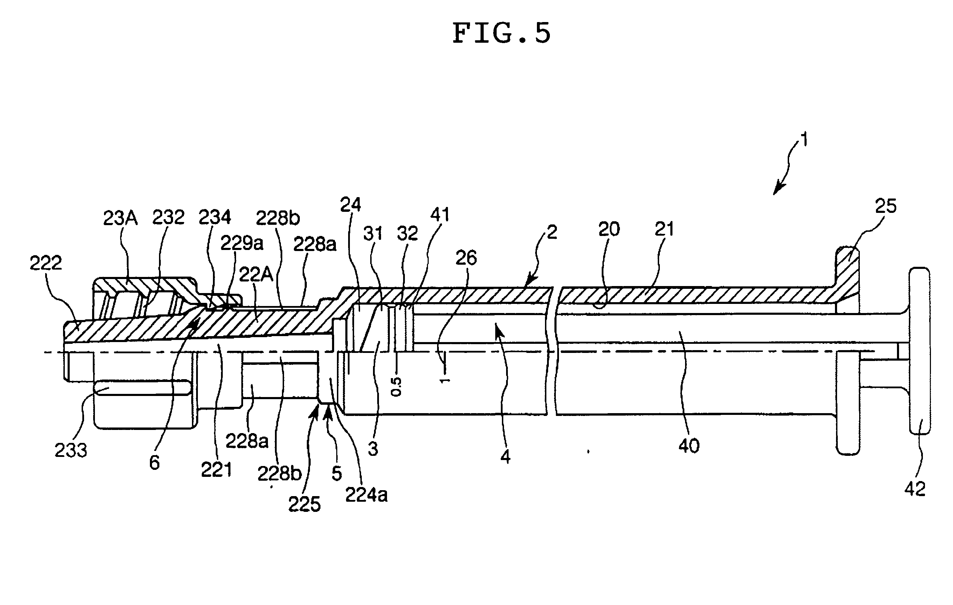

FIG. 1 is a vertical semi-sectional view of a first embodiment of the syringe according to the present invention, and FIGS. 2 to 4 are vertical partly sectional views of a distal end portion of the syringe shown in FIG. 1. Incidentally, for convenience in description, in FIGS. 1 to 4 (and in FIGS. 5 to 8, also), the side of a mouth portion of an outer hollow-cylinder main body will be referred to as "the distal end", and the opposite side will be referred to as "the proximal end".

The syringe 1 shown in FIG. 1 includes an outer hollow cylinder (syringe outer hollow cylinder) 2, a gasket 3 slidable inside the outer hollow cylinder 2, and a pusher (plunger rod) 4 operated for moving the gasket 3. The gasket 3 is connected to the distal end of the pusher 4.

The outer hollow cylinder 2 has an outer hollow-cylinder main body 21 which is in the shape of a bottomed hollow cylinder, and which is integrally provided at its distal end portion with a mouth portion 22 having a smaller diameter than a barrel portion of the outer hollow-cylinder main body 21.

The mouth portion 22 is provided therein with a passage 221 through which a liquid can pass and which is communicated with a bore portion (a space 24 which will be described later) of the outer hollow-cylinder main body 21.

The mouth portion 22 is provided at its distal end portion with a male taper portion 222, and a lock adapter (connection member) 23 is provided on an outer peripheral portion of the mouth portion 22. Incidentally, the configurations of the mouth portion 22 and the lock adapter 23 will be detailed later.

The outer hollow cylinder 2 (outer hollow-cylinder main body 21) is integrally provided with a plate-like flange 25 at the outer periphery of the proximal end thereof. At the time of an operation of moving the pusher 4 relative to the outer hollow cylinder 2 or in other similar situations, the operation can be performed by putting fingers on the flange 25.

Examples of materials for constituting the outer hollow cylinder 2 and the lock adapter 23, which will be described later, include various resins such as polyvinyl chloride, polyethylene, polypropylene, cyclic polyolefins, polystyrene, poly(4-methylpentene-1), polycarbonates, acrylic resins, acrylonitrile-butadiene-styrene copolymer, polyesters such as polyethylene terephthalate, polyethylene naphthalate, etc., butadiene-styrene copolymer, polyamides (e.g. , nylon 6, nylon 6·6, nylon 6·10, nylon 12), ethylene-vinyl alcohol copolymer, polyarylsulfones, polyether sulfones, methacryl-stylene copolymer, polyarylates, and styrene-acrylonitrile copolymer. Among the above examples, preferred are such resins as polypropylene, cyclic polyolefins, polyesters, and poly(4-methylpentene-1), in view of their easy moldability.

Incidentally, it is preferable that the materials constituting the outer hollow cylinder 2 and the lock adapter 23 are substantially transparent, for the purpose of securing visibility of the inside thereof.

In addition, the outer hollow cylinder 2 is provided on its outer peripheral surface with graduations 26 (see FIG. 1). This makes it possible to grasp the amount of the liquid contained in the syringe 1.

In the outer hollow cylinder 2 (the outer hollow-cylinder main body 21), the gasket 3 formed of an elastic material is contained. The gasket 3 is provided at its outer peripheral portion with a plurality of annular projected portions 31, 32 over the entire circumference. The projected portions 31, 32 keep close contact with the inner peripheral surface 20 of the outer hollow cylinder 2 at the time of sliding, whereby gas-tightness (liquid-tightness) can be maintained more securely and, simultaneously, an enhanced slidability can be contrived.

In this embodiment, two projected portions 31, 32 are provided along the longitudinal direction of the gasket 3. Specifically, the projected portions 31, 32 are provided respectively at both proximal end and distal end portions of the gasket 3.

Incidentally, in the present invention, the positions, the number, the sectional shapes and the like of the projected portions 31, 32 are not limited to those shown here.

Besides, the gasket 3 is provided with a hollow portion opening at the proximal end face thereof. In the hollow portion, a head portion of the pusher 4 which will be described later is inserted (fitted).

The material constituting the gasket 3 is not particularly limited. Examples of the material include elastic materials such as various rubber materials such as natural rubber, butyl rubber, isoprene rubber, butadiene rubber, styrene-butadiene rubber, silicone rubbers, fluoro-rubbers, etc., various thermoplastic elastomers such as polyurethane-based ones, plyester-based ones, polyamide-based ones, olefin-based ones, styrene-based ones, etc., and mixtures and the like thereof.

Incidentally, the gasket 3 may have, for example, a configuration in which a core portion (not shown) is formed of a resin material and an elastic material is so disposed as to cover the outer periphery of the core portion. In this case, the hollow portion is formed in the core portion.

Further, the whole part or a part of the gasket 3 may be laminated with a resin. Examples of the resin to be used for the lamination include fluoro-resins such as polytetrafluoroethylene (PTFE), tetrafluoroethyleneperfluoroethylene copolymer (PFA), ethylenetetrafluoroethylene copolymer (ETFE), tetrafluoroethylenehexafluoropropylene copolymer (FEP), etc., high molecular weight polyethylene, and the like.

To the gasket 3 as above, the pusher 4 operated for moving the gasket 3 inside the outer hollow cylinder 2 in the longitudinal direction is connected (attached).

The pusher 4, principally, has a main body portion 40 having a cross section composed of plate pieces arranged in a cross-like shape, and is provided with a plate member 41 at its distal end and with a flange-like (plate-like) finger rest portion 42 at its proximal end, the plate member 41 and the finger rest portion 40 being integral with the main body portion 40. With the finger rest portion 42 pushed by a finger or the like, the pusher 4 is operated to move in the direction toward the distal end.

In addition, the main body portion 40 is provided at its distal end with the head portion (connection portion) to be inserted in the hollow portion of the gasket 3 and to be connected to the gasket 3. With the head portion inserted and fitted in the hollow portion of the gasket 3, the gasket 3 and the pusher 4 are connected to each other.

Examples of the material constituting the pusher 4 include various resins such as polyvinyl chloride, polyethylene, polypropylene, polystyrene, poly(4-methylpentene-1), polycarbonates, acrylic resins, acrylonitrile-butadiene-styrene copolymer, polyesters such as polyethylene terephthalate, polyethylene naphthalate, etc., butadiene-styrene copolymer, and polyamides (e.g., nylon 6, nylon 6·6, nylon 6·10, nylon 12). Among the examples, preferred are such resins as polypropylene, polyesters, and poly(4-methylpentene-1), in view of their easy moldability.

Since the connection between the gasket 3 and the pusher 4 is achieved through the fitting structure, the connection can be carried out assuredly, the gasket 3 and the pusher 4 are prevented from coming off from each other when the pusher 4 is operated, and the pusher 4 can easily be attached to and detached from the gasket 3.

Besides, in the present invention, the connection structure between the gasket 3 and the pusher 4 may be other than the fitting; for example, firm attachment such as adhesion, fusing, etc., screw engagement, and the like may be adopted for the connection.

In this syringe 1, a liquid is contained in a space 24 defined by the gasket 3 and the outer hollow cylinder 2 (inclusive of the passage 221 in the mouth portion 22). The volume of the space 24 is not particularly limited, but the volume when the gasket 3 is moved to be located at the distal end of the inside of the outer hollow cylinder 2 (the dead volume) is preferably not more than 0.1 mL, more preferably not more than 0.07 mL. This ensures that, particularly in the case where an expensive liquid chemical is used as the liquid, the expensive liquid chemical is not wasted and an effective utilization thereof can be contrived.

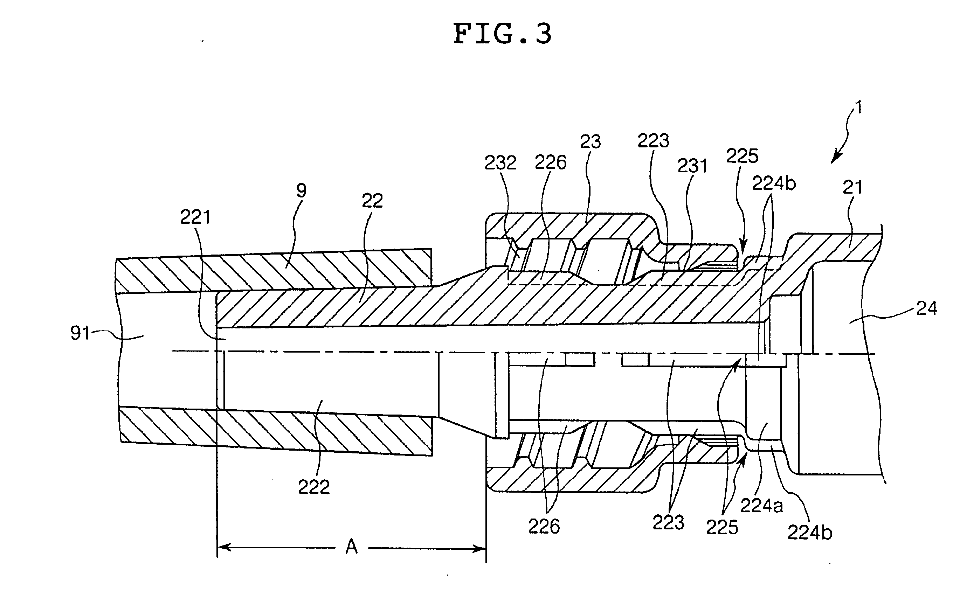

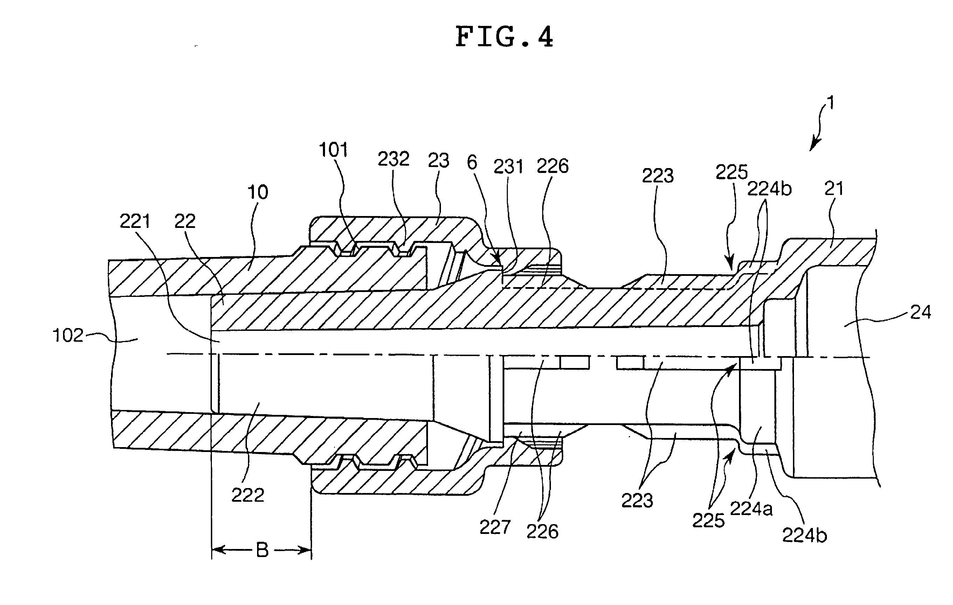

The syringe 1 as above has a distal end portion to which a first female connector as shown in FIG. 3 and a second female connector 10 provided in its outer peripheral surface with a screw groove (female-side screw engagement portion) 101 as shown in FIG. 4 can be alternatively connected. This point (characteristic feature) will be described in detail below.

As has been described above, the mouth portion 22 is provided at the distal end portion of the outer hollow-cylinder main body 21 integrally with the outer hollow-cylinder main body 21, and the lock adapter 23 is provided on the outer peripheral portion of the mouth portion 22.

The mouth portion 22 has at its distal end portion the male taper portion 222 of which the outside diameter and the inside diameter decrease in the direction toward the distal end. The male taper portion 222 is fitted in a bore portion 91 of the first female connector 9 and a bore portion 102 of the second female connector 10.

The gradient of tapering of the male taper portion 222 is not particularly limited, and is, for example, about 4/100 to 8/100, preferably 6/100.

The inside diameter (on average) of the mouth portion 22 is not particularly limited, and is preferably not less than 1.2 mm, more preferably about 1.5 to 2.5 mm. In addition, the length of the mouth portion 22 (in FIG. 2, the length L) is not particularly limited, and is preferably about 16 to 20 mm, more preferably about 17 to 18.5 mm. With the dimensions of the mouth portion 22 set in the above-mentioned ranges, it is possible to reduce the dead volume in the syringe 1.

The lock adapter 23 is relatively rotatable about the mouth portion 22 and is relatively movable along the axial direction of the mouth portion 22. The lock adapter 23 is provided on its inner peripheral surface with a screw thread (male-side screw engagement portion) 232 for screw engagement with the screw groove (female-side screw engagement portion) 101 of the second female connector.

In addition, the lock adapter 23 is provided at its outer peripheral surface with a plurality of ribs (projected portions) 233 along its axial direction. The ribs 233 has the function of preventing fingers or the like from slipping at the time of gripping the lock adapter 23 with the fingers or the like and rotating the lock adapter 23.

The second female connector 10 is connected to a distal end portion of the syringe 1 by the fitting of the male taper portion 222 in its bore portion 102 and the screw engagement of the screw thread 232 of the lock adapter 23 with its screw groove 101 (see FIG. 4).

On the other hand, the first female connector 9 is connected to the syringe 1 by the fitting of the male taper portion 222 in its bore portion 91 (see FIG. 3). Here, if the lock adapter 23 is present at an outer peripheral portion near the male taper portion 222, the lock adapter 23 may obstruct the operation of fitting the male taper portion 222 into the bore portion 91 of the first female connector 9, with the result that the fitting operation cannot be performed assuredly.

In view of this, according to the present invention, the lock adapter 23 can be retracted to a position on the proximal end side such as not to obstruct the operation of fitting the male taper portion 222 into the bore portion 91 of the first female connector 9, and the lock adapter 23 is fixed to the mouth portion 22 when located in the retraction position (the position shown in FIG. 3). This makes it possible to carry out easily and assuredly the operation of fitting the male taper portion 222 into the bore portion 91 of the first female connector 9, i.e., the operation of connecting the first female connector 9 to the distal end portion of the syringe 1.

The lock adapter 23 is provided at the inner peripheral surface of its proximal end portion with an annular projected portion 231 projected toward its center axis, whereas the mouth portion 22 is provided at its outer peripheral surface on the proximal end side with a plurality of (in this embodiment, four) ribs 223 projecting outwards and extending along its axial direction.

At the retraction position, the projected portion 231 (a part of the inner peripheral surface) of the lock adapter 23 is pressed against the ribs 223, whereby the lock adapter 23 is fixed to the mouth portion 22. Namely, the lock adapter 23 is fixed to the mouth portion 22 by fitting when located in the retraction position. This configuration ensures that the operation of fixing the lock adapter 23 to the mouth portion 22 can be performed assuredly and that an operation of canceling the mutual fixation can be easily carried out. In addition, this configuration has the merit that the fixing force (fitting force) for fixing the lock adapter 23 to the mouth portion 22 can be easily regulated, for example, by appropriately setting the number, the width or the like of the ribs 223.

Besides, the plurality of ribs 223 are provided at substantially equal intervals along the circumferential direction of the mouth portion 22. This makes it possible to fix the lock adapter 23 more evenly (uniformly) in the circumferential direction of the mouth portion 22, and to fix the lock adapter 23 and the mouth portion 22 more stably.

Distal end portions of the ribs 223 as above have a height gradually decreasing in the direction toward their distal ends. In other words, the distal end faces of the ribs 223 constitute inclined surfaces. This ensures that, at the time of operation to move the lock adapter 23 to the retraction position, the projected portion 231 of the lock adapter 23 is not caught by edge portions of the ribs 223, so that the operation can be carried out more smoothly.

Incidentally, a configuration may be adopted in which the lock adapter 23 is not fixed at the retraction position.

In addition, the fixing force for fixing the lock adapter 23 at the retraction position to the mouth portion 22 is not particularly limited; for example, the fixing force is set at, or not less than, such a level that the lock adapter 23 does not move under its own weight in the condition where the distal end side of the syringe 1 (the mouth portion 22) is directed vertically downward. This makes it possible to prevent the lock adapter 23 from easily moving from the retraction position.

The protrusion length (length A in FIG. 3) by which the male taper portion 222 protrudes beyond the distal end of the lock adapter 23 when the lock adapter 23 is located in the retraction position is preferably not less than 7.5 mm, more preferably about 7.5 to 9.0 mm. If the protrusion length is too small, it may be impossible to assuredly achieve the fitting of the male taper portion 222 into the bore portion 91 of the first female connector 9, depending on the length (whole length) of the first female connector 9 or the like factors. On the other hand, if the protrusion length is set to exceed the above-mentioned upper limit, the whole length of the syringe 1 would be too large, which is undesirable.

In addition, the syringe 1 has, on the distal end side relative to the retraction position, a rotation permitting position (see FIG. 2) where the lock adapter 23 is rotatable about the mouth portion 22. Specifically, as shown in each figure, the mouth portion 22 has a portion where the ribs 223 are not provided, and, in the condition where the projected portion 231 of the lock adapter 23 is located at this portion, the lock adapter 23 is relatively rotatable about the mouth portion 22.

Furthermore, the syringe 1 has, on the distal end side relative to the rotation permitting position, a distal end side fixation position (see FIG. 4) where the lock adapter 23 is fixed to the mouth portion 22.

The second female connector 10 can be connected to a distal end portion of the syringe 1, by fitting the male taper portion 222 into the bore portion 102 of the second female connector 10 and bringing the screw thread 232 into screw engagement with the screw groove 101 in the condition where the lock adapter 23 is located in the rotation permitting position. Further, the connection can be made firmer, by moving the lock adapter 23 to the distal end side fixation position so as to locate the proximal end of the second female connector 10 on the depth side relative to the lock adapter 23 and thereby to connect the second female connector 10 to the distal end portion of the syringe 1 (see FIG. 4).

At the outer peripheral surface of the mouth portion 22 on the proximal end side relative to the male taper portion 222, a plurality of (in this embodiment, four) ribs 226 projecting outwards are formed along the axial direction.

At the distal end side fixation position, the projected portion 231 (a part of the inner peripheral surface) of the lock adapter 23 is pressed against each of the ribs 226, whereby the lock adapter 23 is fixed to the mouth portion 22. Namely, the lock adapter 23, at the distal end side fixation position, is fixed to the mouth portion 22 through fitting. This configuration makes it possible to perform easily and securely the operation of fixing the lock adapter 23 to the mouth portion 22. In addition, this configuration has the merit that the fixing force (fitting force) for fixing the lock adapter 23 to the mouth portion 22 can be easily regulated, for example, by appropriately setting the number, the width or the like of the ribs 226.

Besides, the plurality of ribs 226 are provided at substantially equal intervals along the circumferential direction of the mouth portion 22. This makes it possible to fix the lock adapter 23 to the mouth portion 22 more evenly (uniformly) in the circumferential direction, and to fix the lock adapter 23 and the mouth portion 22 more stably.

Proximal end portions of the ribs 226 as above have a height gradually decreasing in the direction toward the proximal ends. In other words, the proximal end faces of the ribs 226 constitute inclined surfaces. This ensures that, at the time of operation to move the lock adapter 23 to the distal end side fixation position, the projected portion 231 of the lock adapter 23 is not caught by edge portions of the ribs 226, and the operation can be carried out more smoothly.

In addition, the fixing force for fixing the lock adapter 23 at the distal end side fixation position to the mouth portion 22 is set at, or not less than, such a level that the lock adapter 23 does not move under its own weight in the condition where the proximal end side of the syringe 1 (the mouth portion 22) is directed vertically downwards.

Particularly, the fixing force for fixing the lock adapter 23 at the distal end side fixation position to the mouth portion 22 is preferably set at, or not less then, such a level that the lock adapter 23 is rotated following up to the outer hollow-cylinder main body 21 when the outer hollow-cylinder main body 21 and the second female connector 10 are respectively gripped and the outer hollow-cylinder main body 21 is rotated relative to the second female connector 10 in the condition where the second female connector 10 is connected to the distal end portion of the syringe 1. This makes it possible to easily perform the operation of canceling the connection between the distal end portion of the syringe 1 and the second female connector 10.

The protrusion length (length B in FIG. 4) by which the male taper portion 222 protrudes beyond the distal end of the lock adapter 23 when the lock adapter 23 is located in the distal end side fixation position is preferably not less than 2.1 mm, more preferably about 2.1 to 3 mm. By setting the protrusion length to within this range, the connection of the second female connector 10 to the distal end portion of the syringe 1 can be carried out more assuredly.

The syringe 1 as above has a movement restraining means 5 for restraining the lock adapter 23 from moving to the proximal end side relative to the retraction position, and a detaching preventive means 6 for preventing the lock adapter 23 from coming off (parting) from the mouth portion 22. The movement restraining means 5 and the detaching preventive means 6 will be described below.

The mouth portion 22 has a larger diameter portion 224a having a larger outside diameter at a proximal end portion thereof, and, at the outer peripheral surface of the larger diameter portion 224a, ribs 224b having a height nearly equal to that of the ribs 223 are formed in connection with the ribs 223. Since the outside diameter of the larger diameter portion 224a is greater than the outside diameter of the mouth portion 22, step portions 225 are formed at the boundary portions between the ribs 223 and the ribs 224b. Therefore, when it is intended to move the lock adapter 23 to the proximal end side relative to the retraction position, the proximal end of the lock adapter 223 comes to abut on the step portions 225 and is thereby restrained (inhibited) from moving further to the proximal end side (the side of the outer hollow-cylinder main body 21).

Namely, in this embodiment, the movement restraining means 5 is constituted of the step portions 225.

In addition, on the proximal end side of the male taper portion 222 of the mouth portion 22 (in this embodiment, in the vicinity of the distal ends of the ribs 226), a step portion 227 is provided. Therefore, when it is intended to move the lock adapter 23 to the side of the distal end of the syringe 1 (the distal end side relative to the distal end side fixation position), the distal end of the projected portion 231 of the lock adapter 223 comes to abut on the step portion 227 and is thereby restrained (inhibited) from moving further to the distal end side. As a result, the lock adapter 23 is prevented from coming off from the mouth portion 22.

Namely, in this embodiment, the detaching preventive means 6 is constituted of the step portion 227 and the projected portion 231 of the lock adapter 23. By providing the detaching preventive means 6 as above, it is possible to prevent the second female connector 10 from coming off from the mouth portion 22 together with the lock adapter 23, starting from the condition where the second female connector 10 is connected to the distal end portion of the syringe 1.

In this way, the lock adapter 23 is relatively movable in the axial direction of the mouth portion 22, between the position where its proximal end abuts on the step portions 225 and the position where the distal end of the projected portion 231 abuts on the step portion 227.

The moving distance is not particularly limited, and is preferably not less than 5.4 mm, more preferably about 5.4 to 6.9 mm. If the moving distance is too small, it may be impossible to expose the male taper portion 222 sufficiently from the lock adapter 23 when the lock adapter 23 is located at the retraction position, depending on the length (whole length) of the lock adapter 23 or the like factors. On the other hand, if the protrusion length is set to exceed the above-mentioned upper limit, the whole length of the syringe 1 would be too large, which is undesirable.

Now, one example of the method of using the syringe 1 in this embodiment will be described below.

### [1] In the Case of Connecting the First Female Connector 9

First, the lock adapter 23 is gripped by one hand, the outer hollow-cylinder main body 21 is gripped by the other hand, and the lock adapter 23 is moved toward the outer hollow-cylinder main body 21 in the condition where the outer hollow-cylinder main body 21 is fixed. By this, the lock adapter 23 is moved to the retraction position.

At the retraction position, the projected portion 231 of the lock adapter 23 is pressed against each of the ribs 223, whereby the lock adapter 23 is fixed to the mouth portion 22 by fitting.

Next, under this condition, the first female connector 9 is gripped by one hand, and the male taper portion 222 of the syringe 1 is inserted and fitted into the bore portion 91 of the first female connector 9. By this, the first female connector 9 can be connected to the distal end portion of the syringe 1.

Incidentally, in this instance, since the lock adapter 23 is located in the retraction position, the lock adapter 23 would not obstruct the operation, and the operation of fitting the male taper portion 222 into the bore portion 91 of the first female connector 9 can be performed assuredly.

### [2] In the Case of Connecting the Second Female Connector 10

First, the lock adapter 23 is moved toward the side of the distal end of the syringe 1 so as to press the projected portion 231 against each of the ribs 226, whereby the lock adapter 23 is fixed to the mouth portion 22 by fitting.

Next, the lock adapter 23 is gripped by one hand, whereas the second female connector 10 is gripped by the other hand, and, while inserting the male taper portion 222 of the syringe 1 into the bore portion 102 of the second female connector 10, the lock adapter 23 is rotated. In this instance, since the lock adapter 23 is fixed to the mouth portion 22 by fitting, the rotation of the lock adapter 23 is attended by rotation of the syringe 1 as a whole. As a result, the male taper portion 222 is fitted in the bore portion 102 of the second female connector 10, and the screw thread 232 of the lock adapter 23 makes screw engagement with the screw groove 101 of the second female connector 10, whereby the second female connector 10 can be connected to the distal end portion of the syringe 1.

### <Second Embodiment>

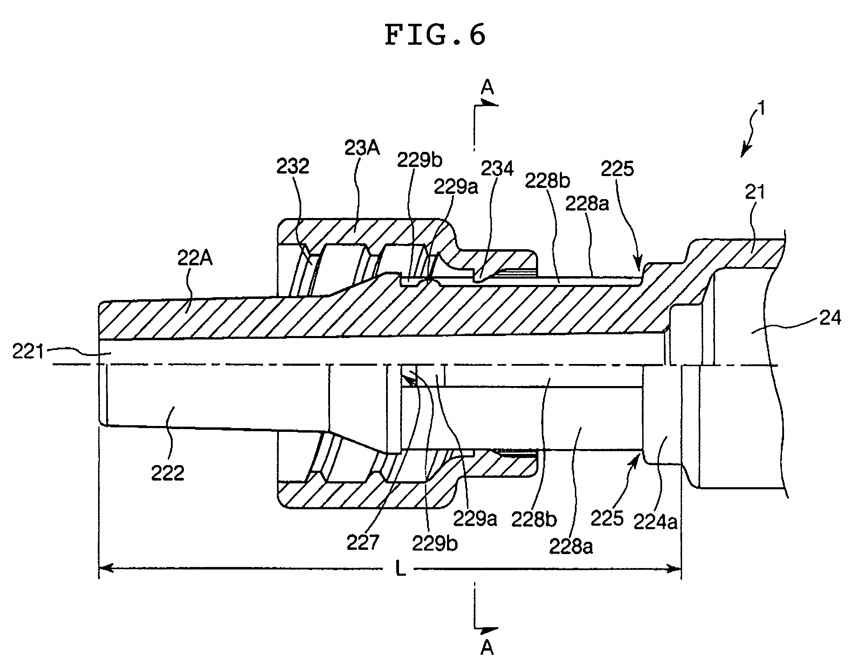

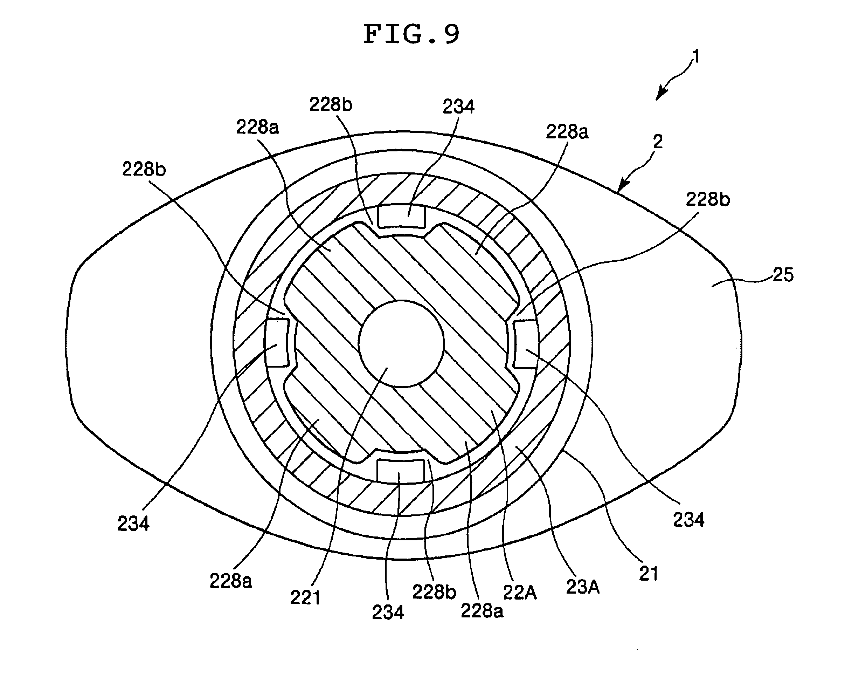

FIG. 5 is a vertical semi-sectional view of a second embodiment of the syringe according to the present invention, FIGS. 6 to 8 are vertical partly sectional views of a distal end portion of the syringe shown in FIG. 5, and FIG. 9 is a sectional view along line A-A of FIG. 6.

Now, the second embodiment of the syringe according to the present invention will be described below referring to the figures. The following description will be centered on differences from the above-described first embodiment, and description of the same points as in the first embodiment will be omitted.

This embodiment is the same as the first embodiment, except that the configurations of a mouth portion of an outer hollow cylinder and a lock adapter are different from those in the first embodiment and that the lock adapter is not fixed to the mouth portion when located in the retraction position.

As shown in FIGS. 5 and 6, the mouth portion 22A of the outer hollow cylinder 2 is provided at its outer peripheral surface with a plurality of (in this embodiment, four) ribs 228a and projected portions 229a.

The ribs 228a are formed to project outwards, along the axial direction of the mouth portion 22A and over the range from a step portion 225 to a step portion 227.

The projected portions 229a are formed to project outwards, on the distal end side of grooves 228b each of which is formed between the two adjacent ribs 228b.

As shown in FIGS. 5 and 6, the lock adapter 23A has a plurality of (in this embodiment, four) pawls 234.

The pawls 234 are formed to project toward the center axis of the lock adapter 23A, at the inner peripheral surface of a proximal end portion of the lock adapter 23A.

As shown in FIG. 9, the lock adapter 23A is provided at the mouth portion 22A so that the pawls 234 are each located between the adjacent ribs 228a, i.e., so that the pawls 234 are contained in the grooves 228b.

This configuration ensures that each of the ribs 228a abuts on the pawls 234 adjacent thereto, to restrain the lock adapter 23A from rotating about the mouth portion 22A. Therefore, the lock adapter 23A is so provided as to be movable along the axial direction of the mouth portion 22A, without rotating about the mouth portion 22A, in the range in which it is movable.

This configuration contributes to simplification of the structure of the outer hollow cylinder 1 (the mouth portion 22A) and to facilitation of the operation on the lock adapter 23A.

As shown in FIG. 9, the four ribs 228a (the pawls 234, also) are provided at substantially equal intervals along the circumferential direction of the mouth portion 22; therefore, the lock adapter 23A can be restrained stably, and the lock adapter 23A can be slid (moved) easily.

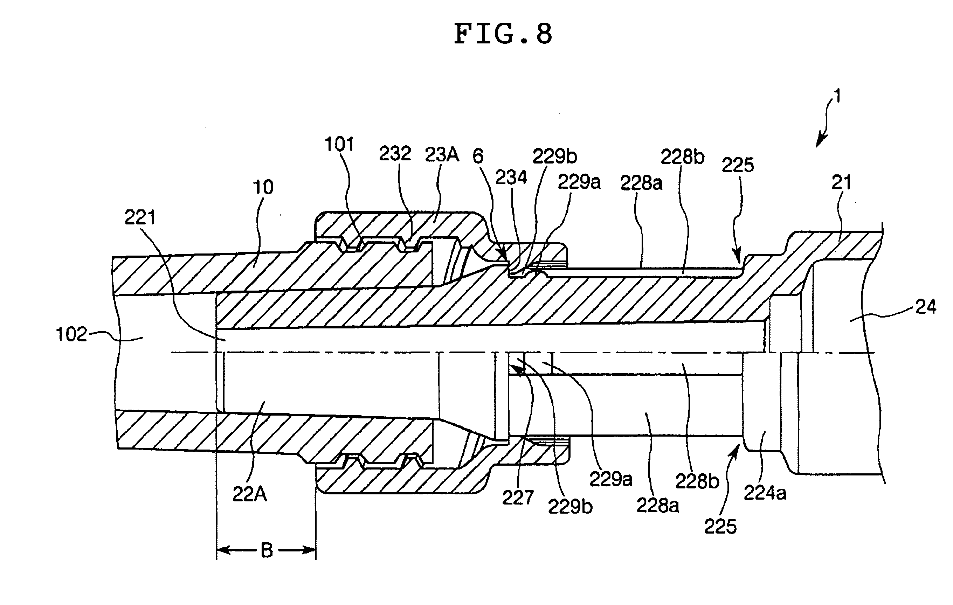

In addition, the syringe 1 has a distal end side fixation position (see FIG. 8) where the lock adapter 23A is fixed to the mouth portion 22A when the lock adapter 23A is located on the distal end side of the mouth portion 22A.

As shown in FIG. 6, the height of the projected portion 229a gradually decreases in the direction toward the distal end and in the direction toward the proximal end. In other words, the distal end face and the proximal end face of the projected portion 229a constitute inclined surfaces, respectively.

This ensures that, at the time when the lock adapter 23A is moved from the proximal end side of the mouth portion 22A to the distal end side fixation position or from the distal end side fixation position to the proximal end side of the mouth portion 22A, the pawls 234 of the lock adapter 23A are not caught by edge portions of the projected portions 229a, and the operations for the movement can be performed more smoothly.

As shown in FIG. 8, at the distal end side fixation position, the pawls 234 are contained in a fixation position space 229b formed between the step portion 227 and the projected portions 229a, whereby the lock adapter 23A is fixed to the mouth portion 22A. Specifically, the lock adapter 23A, at the distal end fixation position, is fixed to the mouth portion 22A because the pawls 234 are restrained, by the step portion 227 and the projected portions 229a, from moving in the axial direction of the mouth portion 22A. In addition, as has been described above, the lock adapter 23A is prevented from rotating about the mouth portion 22A, due to the abutment of each of the ribs 228a on the pawls 234 adjacent thereto.

This configuration makes it possible to carry out easily and assuredly the operation to fix the lock adapter 23A to the mouth portion 22A.

Incidentally, the respective numbers of the ribs 228a (the grooves 228b), the projected portions 229a and the pawls 234 are not limited to four; for example, the numbers may be two or three, or may be five or more.

In addition, the height of the projected portions 229a and the height of the pawls 234 are preferably equivalent to or slightly smaller than the height of the ribs 228a (the depth of the grooves 228b).

Besides, the width of the pawls 234 is preferably equivalent to or slightly smaller than the width of the grooves 228b.

Now, one example of the method of using the syringe 1 in this embodiment will be described below. Like the above description, the following description will be centered on differences from the example of the method of using the syringe 1 in the first embodiment above, and description of the same points as in the above example will be omitted.

### [1] In the Case of Connecting the First Female Connector 9

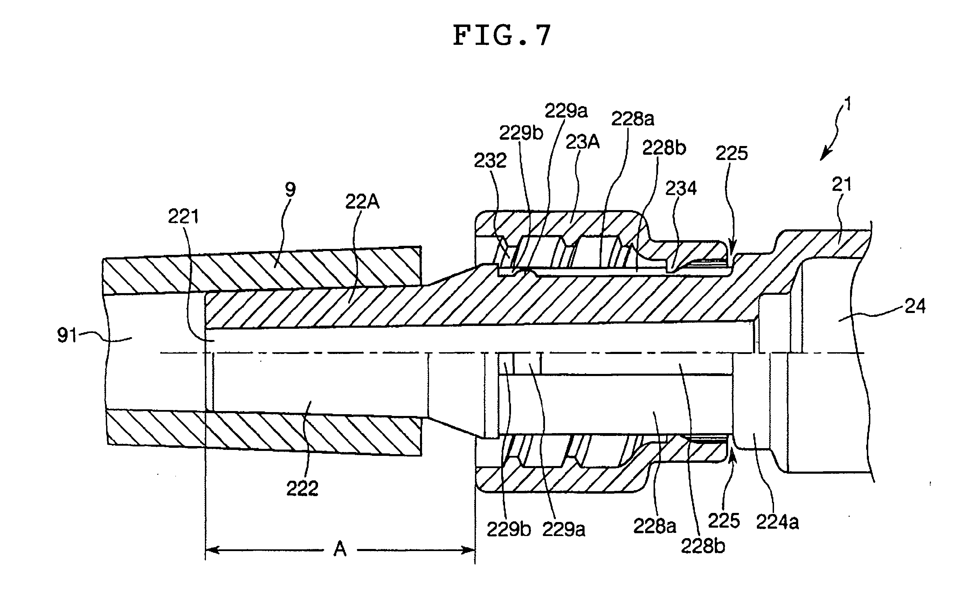

In the same manner as in the first embodiment, when the lock adapter 23A is moved to the retraction position, an outer hollow-cylinder main body 21 is gripped by the other hand, and, under this condition, the lock adapter 23A is fixed by the same hand (see FIG. 7).

Thereafter, the same procedure as in the first embodiment is executed.

### [2] In the Case of Connecting the Second Female Connector 10

First, the lock adapter 23A is moved to the distal end side fixation position, and the lock adapter 23A is fixed to the mouth portion 22A.

Next, the syringe 1 is gripped by one hand, whereas the second female connector 10 is gripped by the other hand, and, while inserting the male taper portion 222 of the syringe 1 into a bore portion 102 of the second female connector 10, the syringe 1 is rotated (see FIG. 8). In this instance, since the lock adapter 23A is integrally fixed to the mouth portion 22A, the rotation of the syringe 1 is attended by rotation of the lock adapter 23A.

Thereafter, the same procedure as in the first embodiment is executed.

While the syringe according to the present invention has been described above referring to the embodiments shown in the figures, the invention is not limited to the above embodiments; for example, the configuration of each of the portions of the syringe can be replaced by an arbitrary one that can display the same or equivalent function as above-mentioned.

In addition, for example, the distal end side fixation position may be provided as required, and may be omitted.

Incidentally, also in the second embodiment, the lock adapter may be fixed to the mouth portion when located in the retraction position, in the same manner as in the first embodiment.

The syringe according to the present invention is applicable to general-use syringes used for giving a drug to a patient, feeding a liquid chemical from an infusion line or a dialysis circuit, mixing of liquid chemicals, etc. In addition, the syringe is applicable also to prefilled syringes in which a liquid chemical is preliminarily placed (contained) in a syringe.

In the case of a prefilled syringe, a liquid chemical is contained, as required, in the outer hollow cylinder of the syringe. The kind of the drug in the liquid chemical is not particularly limited, and examples thereof include sedatives such as diazepam, midazolam, etc., intravenous anesthetics such as propofol, etc., anesthetic analgesics such as fentanyl citrate, morphine hydrochloride, etc., local anesthetics such as mepivacaine hydrochloride, lidocaine hydrochloride, etc., antidepolarizing muscle relaxants such as suxamethonium chloride, pancuronium bromide, etc., catecholamines such as epinephrine, dopamine hydrochloride, dobutamine hydrochloride, etc., vasopressors such as ephedrine hydrochloride, etc., depressors such as nicardipine hydrochloride, chlorpromazine hydrochloride, propranolol hydrochloride, etc., coronary-vessel vasodilators such as isosorbide dinitrate, nitroglycerin, etc., diuretics such as furosemide, aminophylline, etc., antiarrhythmic agents such as atropine sulfate, etc., bronchodilators such as prostaglandin E1 preparation, aminophylline, ephedrine hydrochloride, etc., drugs for peptic ulcer such as famotidine, etc., hormone agents such as nandrolone decanoate, dexamethasone sodium phosphate, betamethasone, human insulin, etc., styptics such as tranexamic acid, etc., blood coagulants such as heparin sodium, etc., antishock agents such as urinastatin, etc., drugs for Parkinson's disease such as biperiden lactate, levodopa, etc., vitamin agents, correction electrolyte liquids, G-CSF preparations, drugs for hepatic diseases, human erythropietin preparations such as epoetin α, etc., drugs for osteoporosis such as elcatonin, etc., synthetic salmon calcitonin preparations, polyvalent antienzymes, drugs for malignant tumor such as paclitaxel, carboplatin, cisplatin, etoposide, etc., antiallergic agents such as chlorpheniramine d-maleate, etc., antibiotic preparations, antitubercular agents, chemotherapic agents, and hepatitis B vaccine.

Now, prior to description of a cap and a method of producing a prefilled syringe provided with the cap according to the present invention, a syringe used with the cap of the present invention mounted thereto will be described below, taking a prefilled syringe as an example.

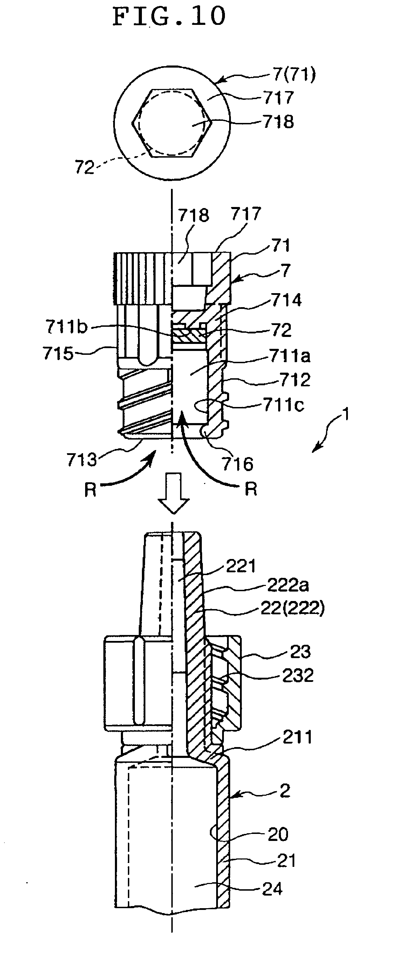

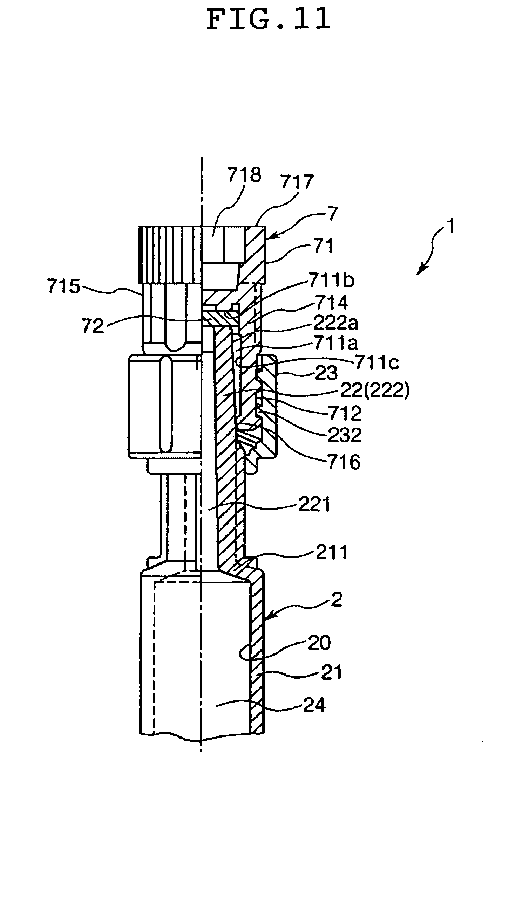

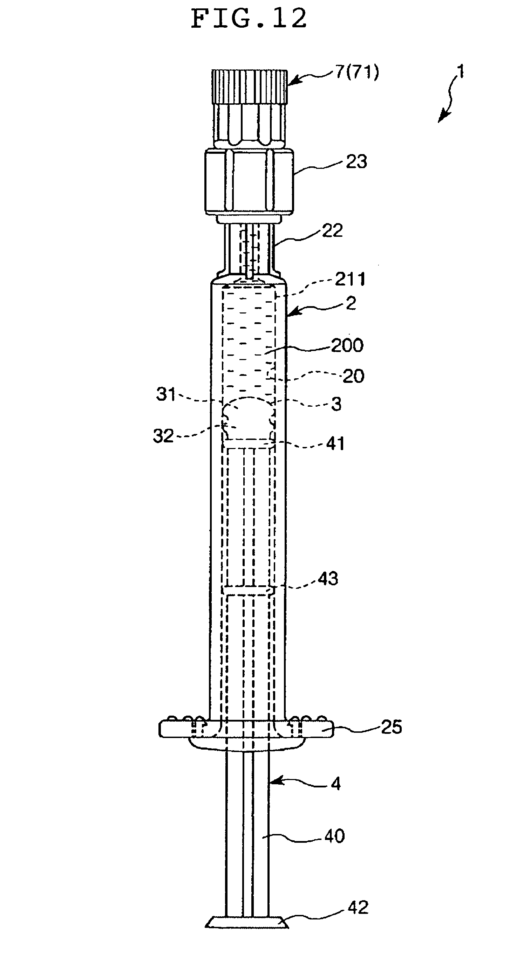

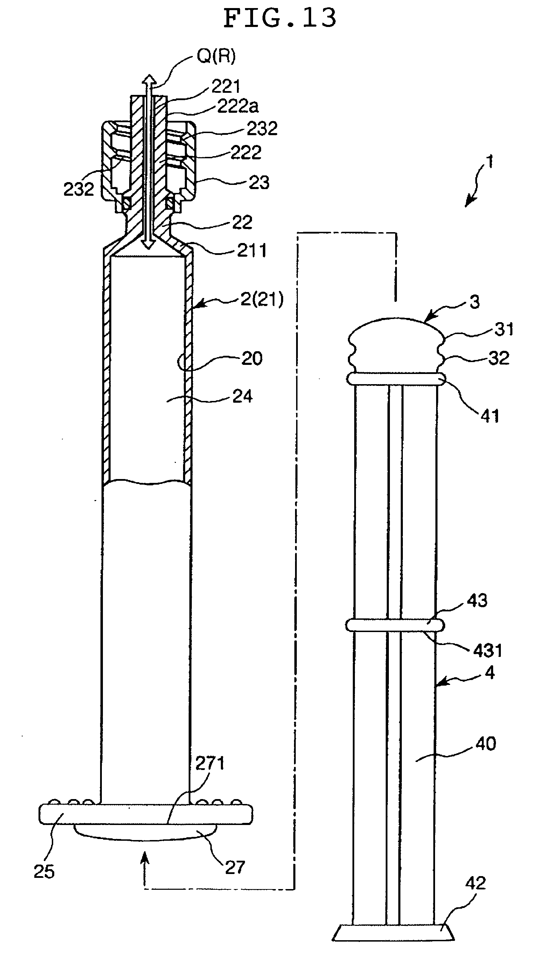

FIG. 13 is a vertical partly sectional view of the prefilled syringe. In the following, for convenience of description, the side of a lock adapter in FIG. 13 (in FIG. 12, also) will be referred to as "the distal end", and the side of a finger rest portion of a syringe main body portion will be referred to as "the proximal end". Incidentally, in FIGS. 10 and 11, the side of a cap will be referred to as "the distal end", and the side of an outer hollow cylinder will be referred to as "the proximal end".

The prefilled syringe (hereinafter referred to simply as "the syringe") 1 shown in FIG. 13 has a liquid preparation 200 (see FIG. 12) preliminarily contained in the inside of the syringe 1. The syringe 1 is composed of a syringe outer hollow cylinder (outer hollow cylinder) 2, a gasket 3 slidable inside the syringe outer hollow cylinder 2, and a plunger (pusher) 4 operated to move the gasket 3.

The outer hollow cylinder 2 is composed of a bottomed hollow cylindrical member having a bottom portion 211.

The outer hollow cylinder 2 is integrally provided with a plate-like flange 25 at the outer periphery of its proximal end. At the time of operating the pusher 4 to move relative to the outer hollow cylinder 2 or in a similar situation, the operation can be performed by putting fingers on the flange 25.

At a central portion of the bottom portion 211 of the outer hollow cylinder 2, a mouth portion 22 having a smaller diameter than a barrel portion (outer hollow-cylinder main body 21) of the outer hollow cylinder 2 and projecting is integrally formed.

The mouth portion 22 is provided therein with a passage 221 through which a fluid Q can pass and which is communicated with a bore portion (bore portion, or space 24) of the outer hollow cylinder 2.

The mouth portion 22 is provided at its distal end portion with a male taper portion 222 of which the outside diameter and the inside diameter gradually decrease in the direction toward the distal end.

On an outer peripheral portion of the mouth portion 22, a hollow cylindrical lock adapter (connection member) 23 is provided so as to be movable along the axial direction of the mouth portion 22. The lock adapter 23 is provided at its inner peripheral surface with a screw thread (male-side screw engagement portion) 232.

A cap 7 according to the present invention can be mounted to the mouth portion 22 of the outer hollow cylinder 2 (syringe 1), by inserting a distal end portion of the mouth portion 22 into a bore portion 711a of a cap main body 71 (the cap 7), which will be described later, and putting a screw groove (female-side screw engagement portion) 712 of the cap main body 71 and a screw thread 232 of the lock adapter 23 into screw engagement with each other.

Examples of materials which can be used to constitute the outer hollow cylinder 2 (inclusive of the lock adapter 23) and the pusher 4, which will be described later, include various hard materials, various glass materials, etc.

Incidentally, it is preferable for the material constituting the outer hollow cylinder 2 to be substantially transparent, for the purpose of securing visibility of the inside.

In addition, the outer hollow cylinder 2 is preferably provided with graduations (not shown) on its outer peripheral surface. This makes it possible to grasp the amount of the liquid preparation 200 contained in the syringe 1.

In the outer hollow cylinder 2 as above, a gasket 3 formed of an elastic material is contained. The gasket 3 is provided at its outer peripheral portion with two annular projected portions 31, 32 at a predetermined interval along the axial direction. The projected portions 31, 32 keep close contact with the inner peripheral surface 20 of the outer hollow cylinder 2 at the time of sliding, whereby gas-tightness (liquid-tightness) is maintained assuredly and, simultaneously, an enhanced slidability can be contrived.

Besides, the gasket 3 is provided with a hollow portion opening at the proximal end face thereof. A head portion of the pusher 4 is inserted (fitted) in the hollow portion.

The pusher 4 is to be operated to move the gasket 3 in the longitudinal direction inside the outer hollow cylinder 2.

The pusher 4, principally, has a main body portion 40 composed of plate pieces arranged in a cross-like shape in cross section, a plate member 41 at the distal end of the main body portion 40, a plate member 43 at an intermediate portion of the main body portion 40, and a flange-like (plate-like) finger rest portion 42 at the proximal end of the main body portion 40, wherein the plate member 41, the plate member 43 and the finger rest portion 42 are formed integrally with the main body portion 40. The pusher 4 is operated to move in the direction toward the distal end, by pushing the finger rest portion 42 with a finger or the like.

In addition, the flange 25 of the outer hollow cylinder 2 is provided with a detaching preventive member 27 which abuts on the plate member 43 when the pusher 4 is operated to move in the direction toward the proximal end. The detaching preventive member 27 is so designed that, when the pusher 4 is operated to move in the direction toward the proximal end, the distal end side surface 271 of the detaching preventive member 27 and the proximal end side surface 431 of the plate member 43 abut on each other in the space 24, thereby restricting the movement of the pusher 4. This prevents the gasket 3 and the pusher 4 from coming off from the outer hollow cylinder 2.

To the mouth portion 22 of the syringe 1 (the outer hollow cylinder 2) as above, the cap 7 according to the present invention is detachably mounted.

Now, the cap and the method of producing the prefilled syringe according to the present invention will be described in detail below, based on the preferred embodiments shown in the accompanying drawings.

First, the cap 7 according to the present invention will be described.

FIGS. 10 and 11 are vertical semi-sectional views of one embodiment of the cap according to the present invention.

The cap 7 shown in FIGS. 10 and 11 is composed of the cap main body 71 and a packing (sealing member) 72.

As shown in FIG. 10, the cap main body 71 is composed of a bottomed hollow cylindrical member, and is provided with the bore portion 711a and the screw groove 712.

The bore portion 711a is roughly cylindrical in shape, and is formed to extend from the proximal end face 713 to the vicinity of an intermediate portion 714.

At the distal end face 711b of the surfaces surrounding the bore portion 711a, the plate-like (disk-like) packing 72 formed of an elastic material is provided in parallel to the distal end face 711b. Incidentally, the diameter of the packing 72 is preferably equivalent to or slightly greater than the diameter of the bore portion 711a. This can prevent the packing 72 from easily coming off from the bore portion 711a (the cap main body 71).

The screw groove 712 is formed in the outer peripheral surface 715 on the proximal end side of the cap main body 71.

In addition, the distal end face 717 of the cap main body 71 is provided with a recessed portion 718 which is roughly regular hexagonal in shape in plan view.

At the time of mounting the cap 7 onto the mouth portion 22, a hexagon wrench (not shown) is fitted into the recessed portion 718. Then, a torque about the axis of the cap 7 (the cap main body 71) is exerted on the hexagon wrench to rotate the cap 7, whereby the screw thread 232 of the lock adapter 23 and the screw groove 712 of the cap main body 71 can be easily and assuredly brought into screw engagement with each other.

Next, the condition where the cap 7 (the cap main body 71) is mounted to the mouth portion 22 of the outer hollow cylinder 2 (this condition will hereinafter be referred to as "the capped condition") will be described below (see FIG. 11).

In the capped condition, the cap 7 is so configured that a part of the inner peripheral surface 711c of the surfaces surrounding the bore portion 711a makes close contact with the male taper portion 222 over the entire circumference.

Specifically, the bore portion 711a (the inner peripheral surface 711c) is provided at its proximal end portion with a smaller diameter portion 716 where the inside diameter of the bore portion 711a is reduced, and the smaller diameter portion 716 is fitted over the mouth portion 22 (the male taper portion 222). This ensures close contact between the smaller diameter portion 716 and a proximal end portion of the mouth portion 22.

Similarly, in the capped condition, the lock adapter 23 is located on the distal end side of the mouth portion 22, and the screw thread 232 of the lock adapter 23 is in screw engagement with the screw groove 712 of the cap 7.

This configuration makes it possible to keep the inside of the bore portion 711a in a hermetically sealed condition. Specifically, the outside surface 222a of the mouth portion 22 (the male taper portion 222) located inside the bore portion 711a can be isolated from the outside of the syringe 1, by the cap 7.

In addition, similarly, in the capped condition, the packing 72 is clamped between the distal end face 711b (end face) of the bore portion 711a and the distal end of the mouth portion 22, to seal the mouth portion 22 (the passage 221) in a liquid-tight (gas-tight) manner. Incidentally, the thickness of the packing 72 is preferably equivalent to or slightly greater than the distance between the distal end face 711b and the distal end of the mouth portion 22. This ensures that the packing 72 in the capped condition is elastically deformed in the thickness direction thereof, whereby the mouth portion 22 can be sealed more assuredly.

This configuration makes it possible to prevent the liquid preparation 200 such as a liquid chemical from leaking out of the mouth portion 22 when the liquid preparation 200 is placed (contained) in the inside of the syringe 1 (in the space 24 in the outer hollow cylinder 2).

Incidentally, the hermetically sealed condition of the inside of the bore portion 711a is maintained by the close contact between the cap main body 71 (the smaller diameter portion 716) and the mouth portion 22 (the male taper portion 222), as has been described above. In this embodiment, the hermetically sealed condition is maintained also by the close contact between the packing 72 and the distal end of the mouth portion 22.

Examples of the material for constituting the cap main body 71 include various resins such as polyvinyl chloride, polyethylene, polypropylene, cyclic polyolefins, polystyrene, poly(4-methylpentene-1), polycarbonates, acrylic resins, acrylonitrile-butadiene-styrene copolymer, polyesters such as polyethylene terephthalate, polyethylene naphthalate, etc., butadiene-styrene copolymer, polyamides (e.g., nylon 6, nylon 6·6, nylon 6·10, nylon 12), ethylene-vinyl alcohol copolymer, polyarylsulfones, polyether sulfones, methacryl-styrene copolymer, polyarylates, styrene-acrylonitrile copolymer, etc. Among these examples, preferred are such resins as polypropylene, cyclic polyolefins, polyesters, and poly(4-methylpentene-1), in view of their easy moldability.

On the other hand, examples of the material for constituting the packing 72 include various rubber materials (particularly, vulcanized ones) such as natural rubber, isoprene rubber, butadiene rubber, styrene-butadiene rubber, nitrile rubber, chloroprene rubber, butyl rubber, acrylic rubber, ethylene-propylene rubber, epichlorohydrin rubber, urethane rubber, silicone rubbers, fluoro-rubbers, etc., various thermoplastic elastomers such as styrene-based ones, polyolefin-based ones, polyvinyl chloride-based ones, polyurethane-based ones, polyester-based ones, polyamide-based ones, polybutadiene-based ones, transpolyisoprene-based ones, fluoro-rubber-based ones, chlorinated polyethylene-based ones, etc., and comparatively soft resin materials such as polyethylene, polyvinyl resins, etc., which can be used either singly or in mixture of two or more of them.

Now, a method of producing (assembling) the prefilled syringe 1 using the cap 7 will be described below.

### <First Embodiment>

FIG. 12 is a side view of the prefilled syringe with the cap shown in FIG. 10 mounted thereto.

The steps for producing the prefilled syringe 1 will be described below.

### [A1] Sterilizing Step (First Step)

First, an outer hollow cylinder 2 and a cap 7 are prepared, and are sterilized respectively.

Incidentally, the sterilization in this instance is not particularly limited, and may be carried out by, for example, autoclave sterilization, gas sterilization using a sterilizing gas such as hydrogen peroxide, EOG, etc., radiation sterilization utilizing irradiation with radioactive rays such as γ-rays, electron beams, etc., or the like. Now, individual cases of using these sterilizing methods will be described below.

### [A1-1] Step Using Autoclave Sterilization (Autoclave Sterilization Step)

The outer hollow cylinder 2 and the cap 7 are placed in an autoclave sterilization system. In this condition, high-pressure water vapor R is supplied into the system to perform sterilization.

In this instance, the high-pressure water vapor R spreads into the bore portion 711a of the cap 7, whereby the cap 7, particularly, the bore portion 711a is sterilized sufficiently (see FIG. 10). In addition, the high-pressure water vapor R sterilizes the surface of the mouth portion 22 sufficiently, and passes through the passage 221 to sufficiently sterilize the inside of the outer hollow cylinder 2, particularly, the inside (the passage 221) of the mouth portion 22 (see FIG. 13).

### [A1-2] Step Using Gas Sterilization (Gas Sterilization Step)

The outer hollow cylinder 2 and the cap 7 are respectively sterilized, in substantially the same manner as in the above-mentioned step [A1-1].

### [A1-3] Step Using Radiation Sterilization (Radiation Sterilization Step)

The outer hollow cylinder 2 and the cap 7 are respectively contained in containing members (wrapping materials), and opening portions of the containing members are sealed to keep the inside in a gas-tight condition. In this condition, irradiation with radioactive rays is conducted to perform radiation sterilization.

### [A2] Capping Step (Second Step)

After the sterilization is finished, the cap 7 and the outer hollow cylinder 2 are transferred into an isolator placed in a sterile environment, and the cap 7 is mounted to the mouth portion 22 of the outer hollow cylinder 2 (see FIG. 11).

In this instance, the above-described configuration ensures that the mouth portion 22 located inside the bore portion 711a can be prevented from making contact with other component parts or the like, and the risk of generation or deposition of foreign matter, particulates or the like at or on the mouth portion 22 can be obviated. Therefore, the outside surface 222a of the mouth portion 22 can be maintained in a sterile condition.

### [A3] Liquid Preparation Placing Step (Third Step)

Thereafter, in a sterile environment, the liquid preparation 200 having been preliminarily subjected to a sterilizing treatment (e.g., filtration sterilization or the like) is placed into the outer hollow cylinder 2 through an opening portion provided at the proximal end of the outer hollow cylinder 2. The amount of the liquid preparation 200 thus placed is set to be about 0.1 to 100 mL, correspondingly to the inside volume of the syringe 1. After the liquid preparation 200 is thus placed, the gasket 3 and the pusher 4 are mounted in position, to complete the prefilled syringe 1 as shown in FIG. 12.

Incidentally, in this condition, the mouth portion 22 (the passage 221) of the outer hollow cylinder 2 is sealed with the packing 72 in a liquid-tight (gas-tight) manner, as has been mentioned above; therefore, the liquid preparation 200 would not lead out of the syringe 1.

By the method as above, the prefilled syringe 1 can be produced in a sterile manner.

Incidentally, examples of the liquid preparation 200 include various liquid chemicals and various diagnostic chemicals such as blood preparations, carbohydrate injections such as grape sugar, etc., electrolyte correction injections such as sodium chloride, potassium lactate, etc., vitamin agents, vaccines, antibiotic injections, contrast media, steroid agents, proteolytic enzyme inhibitors, lipid emulsions, various protein preparations, carcinostatic agents, anesthetics, stimulants, narcotic, etc., and liquids such as distilled water, physiological saline, disinfectants, nutrient agents, fluid foods, alcohols, etc.

### <Second Embodiment>

Now, a second embodiment of the method of producing a prefilled syringe will be described below. The following description will be centered on differences from the first embodiment above, and description of the same points in the first embodiments will be omitted.

The second embodiment is the same as the first embodiment, except that the order of the step of sterilizing the outer hollow cylinder 2 and the cap 7 (sterilising step) and the step of mounting the cap 7 to the mouth portion 22 (capping step) is different.

### [B1] Capping Step (First Step)

First, the outer hollow cylinder 2 and the cap 7 are prepared, and the cap 7 is mounted onto the mouth portion 22 of the outer hollow cylinder 2 (see FIG. 11).

### [B2] Sterilizing Step (Second Step)

In this condition (the capped condition), the outer hollow cylinder 2 (inclusive of the cap 7) is sterilized.

Incidentally, the sterilization in this instance is conducted by, for example, radiation sterilization using radioactive rays such as γ-rays and electron beams. In the case of using the radiation sterilization, the same step as the above-mentioned step [A1-3] is conducted.

In this instance, the above-mentioned configuration makes it possible to attain the same effects as above-mentioned.

### [B3] Liquid Preparation Placing Step (Third Step)

The same step as the above-mentioned step [A3] is carried out.

By this method, the prefilled syringe 1 can be produced in a sterile manner, in the same way as in the first embodiment.

While the cap and the method of producing a prefilled syringe according to the present invention have been described above referring to the embodiments shown in the figures, the present invention is not limited to the above embodiments, and each of the portions constituting the cap may be replaced by one having an arbitrary configuration which can display the same function as above. Besides, an arbitrary component or components may be added.

In addition, the cap according to the present invention can be used for other uses than the production of the prefilled syringe comprising the lock adapter described above. For example, in the case of connecting tubes to each other, connectors designed for fitting a male connector into a female connector are ordinarily used, and, in some cases, a lock adapter having the above-mentioned structure may be used for the male connector. In such a case, it is preferable that the portion, to be inserted in the female connector, of the male connector is not exposed to the external environment until the connection is performed. The cap according to the present invention can be used, in such a case, for the purpose of sealing the distal end opening of the male connector and, simultaneously, keeping the male connector in a sterile condition.

### Industrial Applicability

With the syringe according to the present invention, it is possible to connect easily and assuredly both a female connector having a female-side screw engagement portion at its outer peripheral surface and a female connector not having the female-side screw engagement portion.

In addition, by appropriately setting the moving distance of the lock adapter relative to the mouth portion, the protrusion length by which the male taper portion protrudes beyond the lock adapter when the lock adapter is located in the retraction position, or the like, it is possible to perform easily and assuredly the operation of fitting the male taper portion into the bore portion of the first female connector and, as a result, to connect the first female connector more assuredly.

Besides, by providing the distal end side fixation position, it is possible to fix the second female connector more firmly.

With the cap according to the present invention, it is possible to sufficiently sterilize the individual portions of the syringe outer hollow cylinder and to keep the surroundings of the mouth portion of the syringe outer hollow cylinder having been sterilized in a sterile condition, in the production of the prefilled syringe comprising the lock adapter.

## Claims

1. A syringe having a distal end portion to which a first female connector and a second female connector provided on an outer peripheral surface thereof with a female-side screw engagement portion can be alternatively connected, said syringe comprising:
a mouth portion which is provided at a distal end portion thereof with a male taper portion to be fitted in a bore portion of said first female connector and a bore portion of said second female connector and which is provided therein with a passage for permitting a liquid to pass therethrough; and
a lock adapter provided at an outer peripheral portion of said mouth portion, said lock adapter being relatively movable in the axial direction of said mouth portion and being provided on an inner peripheral surface thereof with a male-side screw engagement portion for making screw engagement with said female-side screw engagement portion; wherein
said lock adapter can be retracted to a retraction position on the proximal end side at the time of an operation of fitting said male taper portion into said bore portion of said first female connector.

2. A syringe having a distal end portion to which a first female connector and a second female connector provided on an outer peripheral surface thereof with a female-side screw engagement portion can be alternatively connected, said syringe comprising:
a mouth portion which is provided at a distal end portion thereof with a male taper portion to be fitted in a bore portion of said first female connector and a bore portion of said second female connector and which is provided therein with a passage for permitting a liquid to pass therethrough; and
a lock adapter provided at an outer peripheral portion of said mouth portion, said lock adapter being relatively rotatable about said mouth portion, being relatively movable along the axial direction of said mouth portion, and being provided on an inner peripheral surface thereof with a male-side screw engagement portion for making screw engagement with said female-side screw engagement portion; wherein
said lock adapter can be retracted to a retraction position on the proximal end side at the time of an operation of fitting said male taper portion into said bore portion of said first female connector.

3. The syringe as set forth in claim 1 or 2, wherein said male taper portion protrudes beyond the distal end of said lock adapter by not less than 7.5 mm when said lock adapter is located in said retraction position.

4. The syringe as set forth in any of claims 1 to 3, having a distal end side fixation position where said lock adapter is fixed to said mouth portion when said lock adapter is located on the distal end side of said mouth portion.

5. The syringe as set forth in claim 4, having a rotation permitting position where said lock adapter is relatively rotatable about said mouth portion, on the distal end side relative to said retraction position, wherein
said distal end side fixation position is located on the mouth portion distal end side relative to said rotation permitting position.

6. The syringe as set forth in claim 4 or 5, wherein said male taper portion protrudes beyond the distal end of said lock adapter by not less than 2.1 mm when said lock adapter is located in said distal end side fixation position.

7. The syringe as set forth in any of claims 1 to 6, wherein said lock adapter is relatively movable by not less than 5.4 mm along the axial direction of said mouth portion.

8. The syringe as set forth in any of claims 1 to 7, wherein the inside diameter (on average) of said mouth portion is not less than 1.2 mm.

9. The syringe as set forth in any of claims 1 to 8, wherein the length of said mouth portion is in the range of 16 to 20 mm.

10. The syringe as set forth in any of claims 1 to 9, comprising an outer hollow cylinder provided at a distal end portion thereof with said mouth portion and said lock adapter, and a gasket slidable in said outer hollow cylinder, wherein
the volume of a space defined by said outer hollow cylinder and said gasket when said gasket is located at the distal end of the inside of said outer hollow cylinder is not more than 0.1 mL.

11. A cap to be mounted to a mouth portion of a syringe outer hollow cylinder, said syringe outer hollow cylinder comprising said mouth portion projectingly formed at the distal end of said syringe outer hollow cylinder and provided at a distal end portion thereof with a male taper portion, and a lock adapter provided at an outer peripheral portion of said mouth portion, said lock adapter being relatively movable along the axial direction of said mouth portion and being provided on an inner peripheral surface thereof with a male-side screw engagement portion, said cap comprising:
a bottomed hollow-cylindrical cap main body comprising a bore portion, and a female-side screw engagement portion formed on an outer peripheral portion of said cap main body for screw engagement with said male-side screw engagement portion; and
a packing formed of an elastic material and provided in said bore portion of said cap main body; wherein
at least a part of the inner peripheral surface of said bore portion makes close contact with said male taper portion over the entire circumference when said cap main body is mounted to said mouth portion.

12. The cap as set forth in claim 11, wherein when the inner peripheral surface of said bore portion makes close contact with said male taper portion, said packing is clamped between an end face of said bore portion and the distal end of said mouth portion to seal said mouth portion in a liquid-tight manner.

13. A method of producing a prefilled syringe comprising: a syringe outer hollow cylinder having a mouth portion projectingly formed at the distal end of said syringe outer hollow cylinder and provided at a distal end portion thereof with a male taper portion, and a lock adapter provided at an outer peripheral portion of said mouth portion, said lock adapter being relatively movable along the axial direction of said mouth portion and being provided on an inner peripheral surface thereof with a male-side screw engagement portion; a cap as set forth in claim 11 or 12 which is mounted to said mouth portion; and a liquid preparation filling said syringe outer hollow cylinder; said method comprising the steps of:
sterilizing said syringe outer hollow cylinder and said cap;
then mounting said cap to said mouth portion in a sterile environment; and
thereafter feeding said liquid preparation into said syringe outer hollow cylinder in a sterile environment.

14. A method of producing a prefilled syringe comprising: a syringe outer hollow cylinder having a mouth portion projectingly formed at the distal end of said syringe outer hollow cylinder and provided at a distal end portion thereof with a male taper portion, and a lock adapter provided at an outer peripheral portion of said mouth portion, said lock adapter being relatively movable along the axial direction of said mouth portion and being provided on an inner peripheral surface thereof with a male-side screw engagement portion; a cap as set forth in claim 11 or 12 which is mounted to said mouth portion; and a liquid preparation filling said syringe outer hollow cylinder; said method comprising the steps of:
mounting said cap to said mouth portion and performing sterilization under this condition; and
thereafter feeding said liquid preparation into said syringe outer hollow cylinder in a sterile environment.
